Europäisches Patentamt

European Patent Office

Office européen des brevets

(19)

(11) Publication number: **0 011 401**
**B1**

(12) **EUROPEAN PATENT SPECIFICATION**

(45) Date of publication of patent specification: **01.06.83**

(21) Application number: **79302305.2**

(22) Date of filing: **23.10.79**

(51) Int. Cl.³: **C 07 C 85/00,**
**C 07 C 87/14,**
**C 07 C 120/00,**
**C 07 C 121/34,**
**C 07 C 27/22**

(54) Process for the production of hexamethylenediamine.

(30) Priority: **08.11.78 GB 4373278**

(43) Date of publication of application:
**28.05.80 Bulletin 80/11**

(45) Publication of the grant of the patent:
**01.06.83 Bulletin 83/22**

(84) Designated Contracting States:
**BE DE FR GB IT NL**

(56) References cited:
**US - A - 3 520 914**

(73) Proprietor: **IMPERIAL CHEMICAL INDUSTRIES PLC**
**Imperial Chemical House Millbank**
**London SW1P 3JF (GB)**

(72) Inventor: **Wright, Donald**
**9 Connaught Road**
**Nunthorpe Middlesbrough, Cleveland (GB)**

(74) Representative: **Taylor, Michael et al,**
**Imperial Chemical Industries PLC Legal**
**Department: Patents Thames House North**
**Millbank**
**London SW1P 4QG (GB)**

Courier Press, Leamington Spa, England.

Process for the production of hexamethylenediamine

The present invention relates to a process for the production of hexamethylenediamine.

In our British patent No. 1,497,046 we have described a process for the manufacture of cyano-substituted aliphatic carboxylic esters in which an alkenenitrile having at least 3 carbon atoms in the alkene residue is reacted with carbon monoxide and an alcohol under pressure in the presence of a catalyst which is preferably a known carbonylation catalyst e.g. a transition metal.

The alkenenitriles which are singled out in British patent No. 1,497,046 as being particularly useful in the process therein described are those of formula $C_nH_{2n-1}CN$ where n is 4 or 5 e.g. 3-pentenenitrile which, on carbonylation with methanol for example, gives methyl $\delta$-cyanovalerate said to be convertible to adiponitrile by reaction with ammonia in the presence of a dehydrating catalyst, the adiponitrile in turn being capable of hydrogenation to hexamethylene diamine which is an important polymer intermediate.

US Patent No. 3,520,914 describes a process for the hydroformylation of acrylonitrile and its alkyl-substituted derivatives using a conventional hydroformylation catalyst e.g. a cobalt catalyst. The products of the process are 2-hydroxymethylalkane nitriles which are said to be convertible to dicarboxylic acids, amino alcohols or amino acids.

The process described in British patent No. 1,497,046 takes two stages to convert the cyano ester to hexamethylene diamine. We have now devised a process for the production from an alkenenitrile of a cyano-substituted product which may be converted in one step to hexamethylene diamine and under milder conditions than those required by the process of the British patent.

Accordingly, the present invention is a process for the production of hexamethylene diamine characterised in that

(a) 3-pentenenitrile is reacted with carbon monoxide and hydrogen under pressure in the presence of a cobalt catalyst to produce a cyano-substituted aldehyde and/or alcohol and

(b) the cyano-substituted aldehyde and/or alcohol is converted to hexamethylenediamine by hydrogenation with molecular hydrogen in the presence of ammonia and a hydrogenation catalyst.

3-Pentenenitrile which is the starting material for the process of the present invention may conveniently be obtained by the hydrocyanation of butadiene e.g. as described in our British patent No. 1,429,651.

The cobalt catalyst is preferably used in the process in the form of one of its compounds particularly as the carbonyl or carboxylate. The carboxylate may be aliphatic or aromatic in nature, e.g. cobalt benzoate or cobalt naphthenate or a cobalt salt of a $C_1$ to $C_{25}$ alkanoic acid such as cobalt acetate or cobalt stearate. The cobalt is present in the reaction in catalytic amounts, e.g. 1 to 2,000 preferably 50 to 500 parts per million. A biphyllic trivalent phosphorus-containing ligand may also be included in the reaction medium. The ligand may be a phosphite, phosphonite or phosphinite but is preferably a phosphine particularly a trialkylphosphine in which the alkyl groups each contain 1 to 6 carbon atoms. The presence of the ligand tends to produce a more linear cyano-aldehyde and/or cyano-alcohol than is produced in its absence.

The hydroformylation process (a) is carried out at an elevated pressure which is suitably provided by the carbon monoxide and hydrogen preferably the pressure is 25 to 500 bars more preferably 50 to 300 bars. The mole ratio of carbon monoxide to hydrogen is preferably 1:1 but may vary in the range 1:3 to 3:1.

The temperature under which the hydroformylation process (a) is carried out suitably lies in the range 50° to 300°C particularly 100° to 200°C.

The hydroformylation process (a) may conveniently be carried out in the presence of a solvent which should be liquid under the reaction conditions and which should be chemically inert with respect to the reactants. Thus, preferred solvents are hydrocarbon in nature e.g. saturated aliphatic hydrocarbons such as hexane or cyclohexane or aromatic hydrocarbons e.g. benzene, toluene or xylene. Alternatively the solvent may be a ketone e.g. acetone or an ether or ester.

The product of the process may be the aldehyde, alcohol or a mixture of the two. For subsequent conversion to the diamine it is preferred that the aldehyde level be as high as possible. The cyanoaldehyde or cyano-alcohol or mixture of the two is reductively aminated to the corresponding diamine by means of molecular hydrogen and ammonia using a hydrogenation catalyst.

The reductive amination may be carried out under conditions conventional for this type of reaction. Thus the catalyst may be a copper or nickel catalyst e.g. "COPEL" and the reaction may be carried out at elevated temperature and pressure e.g. 100° to 600°C and 5 to 500 bar. A solvent may be used if desired, particularly one of the hydrocarbon solvents listed above.

The invention will now be described with reference to the following Examples:

Example 1

3-Pentenenitrile 8.4 g (0.104 moles) in acetone (30 ml) was heated in an autoclave at 120°C and at 200 bar pressure (CO:H$_2$ ratio 1:1) for 3 hours at a cobalt concentration of 300 ppm. Distillation of the product at 1 mm pressure gave 9.5 g of the cyano valeraldehyde bp 72—76°C. This consisted of a 1:1:1 mixture of the $\beta$, $\gamma$ and $\delta$-cyanoaldehydes.

Example 2

3-pentenenitrile 21 g (0.26 mole) in toluene (20 ml) containing tri-n-butyl phosphine (0.54 g) was heated in an autoclave at 170°C and 120 bar pressure (CO:H$_2$ ratio 1:1) for 1 hour at a cobalt concentration of 1,500 ppm. Distillation at 1 mm pressure gave 2 g of the cyano valeraldehyde mixture.

Example 3

3-pentenenitrile 10 mls (0.10 mole) in toluene (30 mls) was heated in an autoclave at 170°C and 250 bar working pressure for 2 hours at a cobalt concentration of 400 ppm. Analysis of the product showed a selectivity to $\delta$-cyanovaleraldehyde of 65% at 60% conversion.

Example 4

Product from the preceding Examples consisting of a mixture of cyanovaleraldehydes (5.2 g, 0.047 mole) which contained 60% of $\delta$-cyanovaleraldehyde was placed in an autoclave with a solution of ammonia (8.4 g, 0.49 mole) in methanol (23 g) and Raney nickel catalyst (0.25 g). The autoclave was heated at 100°C for 2 hours under a hydrogen pressure of 140 atmospheres. Gas chromatographic analysis of the product showed a 25% conversion of the $\delta$-cyanovaleraldehyde to hexamethylene diamine.

## Claims

1. A process for the production of hexamethylene diamine characterised in that

(a) 3-pentenenitrile is reacted with carbon monoxide and hydrogen under pressure in the presence of a cobalt catalyst to produce a cyano-substituted aldehyde and/or alcohol and,
(b) the cyano-substituted aldehyde and/or alcohol is converted to hexamethylenediamine by hydrogenation with molecular hydrogen in the presence of ammonia and a hydrogenation catalyst.

2. A process according to Claim 1 characterised in that the cobalt catalyst in (a) is cobalt carbonyl or cobalt carboxylate e.g. cobalt benzoate, cobalt naphthenate or a cobalt salt of a C$_1$ to C$_{25}$ alkanoic acid.
3. A process according to either of the preceding claims characterised in that the cobalt in (a) is present in an amount of 1 to 2000 preferably 50 to 500 ppm of the reaction medium.
4. A process according to any one of the preceding claims characterised in that in (a) a biphyllic trivalent phosphorus-containing ligand is present e.g. a trialkylphosphine in which the alkyl groups each contain 1 to 6 carbon atoms.
5. A process according to any one of the preceding claims characterised in that the pressure in (a) is 25 to 500 bars preferably 50 to 300 bars.
6. A process according to any one of the preceding claims characterised in that the temperature in (a) lies in the range 50° to 300°C preferably 100° to 200°C.
7. A process according to any one of the preceding claims characterised in that the mole ratio of carbon monoxide to hydrogen in (a) lies between 1:3 and 3:1 and is preferably 1:1.
8. A process according to any one of the preceding claims characterised in that a solvent is present in (a) e.g. a saturated aliphatic hydrocarbon, an aromatic hydrocarbon or a ketone, ether or ester solvent.
9. A process according to any one of the preceding claims characterised in that the hydrogenation (b) is carried out at 100—600°C and 50 to 500 bar pressure in the presence of a copper or nickel hydrogenation catalyst.

## Revendications

1. Procédé de préparation de l'hexaméthylènediamine, caractérisé en ce que

(a) le 3-pentènenitrile est mis à réagir avec le monoxyde de carbone et 'hydrogène en présence d'un catalyseur au cobalt pour la production d'un aldéhyde et/ou alcool cyano-substitué, et
(b) l'aldéhyde et/ou alcool cyano-substitué est converti en hexaméthylènediamine par hydrogénation au moyen d'hydrogène moléculaire en présence d'ammoniac et d'un catalyseur d'hydrogénation.

2. Procédé suivant la revendication 1, caractérisé en ce que le catalyseur au cobalt en (a) est le cobalt-carbonyle ou un carboxylate de cobalt, par exemple le benzoate de cobalt, le naphténate de cobalt ou un sel de cobalt d'un acide alconoïque en C$_1$ à C$_{25}$.
3. Procédé suivant l'une quelconque des revendications précédentes, caractérisé en ce que le cobalt en (a) est présent en une quantité de 1 à 2.000 et de préférence de 50 à 500 ppm du milieu de réaction.
4. Procédé suivant l'une quelconque des revendications précédentes, caractérisé en ce qu'en (a) un ligande contenant du phosphore trivalent biphylle est présent, par exemple une trialcoylphosphine dont les radicaux alcoyle comptent chacun 1 à 6 atomes de carbone.
5. Procédé suivant l'une quelconque des revendications précédentes, caractérisé en ce que la pression en (a) est de 25 à 500 bars et de préférence de 50 à 300 bars.
6. Procédé suivant l'une quelconque des revendications précédentes, caractérisé en ce que la température en (a) tombe dans l'intervalle de 50 à 300°C et de préférence de 100 à 200°C.

7. Procédé suivant l'une quelconque des revendications précédentes, caractérisé en ce que le rapport molaire du monoxyde de carbone à l'hydrogène en (a) tombe entre 1:3 et 3:1 et est de préférence de 1:1.

8. Procédé suivant l'une quelconque des revendications précédentes, caractérisé en ce qu'un solvant est présent en (a), par exemple un hydrocarbure aliphatique saturé, un hydrocarbure aromatique ou une cétone, un éther ou un ester.

9. Procédé suivant l'une quelconque des revendications précédentes, caractérisé en ce que l'hydrogénation (b) est exécutée à 100—600°C sous une pression de 50 à 500 bars en présence d'un catalyseur d'hydrogénation au cuivre ou au nickel.

**Patentansprüche**

1. Verfahren zur Herstellung von Hexamethylendiamin, dadurch gekennzeichnet, daß

(a) 3-Pentennitril under Druck in Gegenwart eines Cobalt-Katalysators mit Kohlenmonoxid und Wasserstoff umgesetzt wird, wobei ein cyanosubstituierter Aldehyd und/oder Alkohol hergestellt wird, und

(b) der cyanosubstituierte Aldehyd und/oder Alkohol durch Hydrierung mit molekularem Wasserstoff in Gegenwart von Ammoniak und eines Hydrierungskatalysators in Hexamethylendiamin umgewandelt wird.

2. Verfahren nach Anspruch 1, dadurch gekennzeichnet, daß der Cobalt-Katalysator in Stufe (a) Cobaltcarbonyl oder Cobaltcarboxylat, z.B. Cobaltbenzoat, Cobaltnaphthenat oder ein Cobaltsalz einer $C_1$- bis $C_{25}$-Alkansäure, ist.

3. Verfahren nach einem der vorhergehenden Ansprüche, dadurch gekennzeichnet, daß das Cobalt in Stufe (a) in einer Menge von 1 bis 2000 Teilen, vorzugsweise 50 bis 500 Teilen, pro 1 Million Teile des Reaktionsmediums vorliegt.

4. Verfahren nach einem der vorhergehenden Ansprüche, dadurch gekennzeichnet, daß in Stufe (a) ein biphiler, dreiwertigen Phosphor enthaltender Ligand, z.B. ein Trialkylphosphin, in dem die Alkylgruppen jeweils 1 bis 6 Kohlenstoffatome enthalten, vorhanden ist.

5. Verfahren nach einem der vorhergehenden Ansprüche, dadurch gekennzeichnet, daß der Druck in Stufe (a) 25 bis 500 bar, vorzugsweise 50 bis 300 bar, beträgt.

6. Verfahren nach einem der vorhergehenden Ansprüche, dadurch gekennzeichnet, daß die Temperatur in Stufe (a) in dem Bereich von 50° bis 300°C, vorzugsweise von 100° bis 200°C, liegt.

7. Verfahren nach einem der vorhergehenden Ansprüche, dadurch gekennzeichnet, daß das Molverhältnis von Kohlenmonoxid zu Wasserstoff in Stufe (a) zwischen 1:3 und 3:1 liegt und vorzugsweise 1:1 beträgt.

8. Verfahren nach einem der vorhergehenden Ansprüche, dadurch gekennzeichnet, daß in Stufe (a) ein Lösungsmittel, z.B. ein gesättigter, aliphatischer Kohlenwasserstoff, ein aromatischer Kohlenwasserstoff oder ein Keton, Ether, oder Ester, vorhanden ist.

9. Verfahren nach einem der vorhergehenden Ansprüche, dadurch gekennzeichnet, daß die Hydrierung (b) bei 100° bis 600°C und unter einem Druck von 50 bis 500 bar in Gegenwart eines Kupfer- oder Nickel-Hydrierungskatalysators durchgeführt wird.